Europäisches Patentamt

(19) European Patent Office

Office européen des brevets

(11) Veröffentlichungsnummer: **0 099 074**
**A2**

(12) # EUROPÄISCHE PATENTANMELDUNG

(21) Anmeldenummer: 83106648.5

(22) Anmeldetag: 07.07.83

(51) Int. Cl.³: **A 61 L 15/01**
**A 61 L 15/03, A 61 K 31/74**
**A 61 K 31/785**

(30) Priorität: 14.07.82 DE 3226754

(43) Veröffentlichungstag der Anmeldung:
25.01.84 Patentblatt 84/4

(84) Benannte Vertragsstaaten:
AT BE CH DE FR GB IT LI LU NL SE

(71) Anmelder: SCHERING AKTIENGESELLSCHAFT Berlin
und Bergkamen
Müllerstrasse 170/178 Postfach 65 03 11
D-1000 Berlin 65(DE)

(72) Erfinder: Wündisch, Karl
c/o RA Krüger Mommsenstrasse 65
D-1000 Berlin 12(DE)

(72) Erfinder: Zimmermann, Ingfried, Dr.
Gollanczstrasse 28 c
D-1000 Berlin 28(DE)

(54) Mittel zur Behandlung von Wunden.

(57) Es wird ein Mittel zur Behandlung von Wunden, beschrieben, daß dadurch gekennzeichnet ist, daß es 2 bis 20
Gewichtsprozent eines Pfropf-Copolymerisats aus Stärke
und hydrolysiertem Polyacrylnitril, dessen Carboxylgruppen-Wasserstoffe zu 5 bis 90 % durch Aluminium substituiert
sind, in einer physiologisch unbedenklichen, 1 bis 3 Tenside
enthaltenden lipophilen Flüssigkeit oder Paste suspendiert
sind.

EP 0 099 074 A2

Croydon Printing Company Ltd.

Mittel zur Behandlung von Entzündungen von Wunden sind vorbekannt. So findet neben den konventionellen textilen Verbänden unter anderem ein Produkt auf Dextran-Basis Anwendung bei der Behandlung von Wunden (US-Patent 4,225,580). Dieses Material hat aber den Nachteil, daß es relativ schwierig zu applizieren ist, und insbesondere den Nachteil, daß es relativ schwierig aus der Wunde zu entfernen ist, da die kleinen Dextrankörner leicht mit den Wundrändern verkleben.

Es wurde nun gefunden, daß Mittel zur Wundbehandlung die 2 bis 20 Gewichtsprozent eines Pfropf-Copolymerisats aus Stärke und hydrolysiertem Polyacrylnitril, dessen Carboxylgruppen-Wasserstoffe zu 5 bis 90 % durch Aluminium substituiert sind, welche in einer physiologisch unbedenklichen, 1 bis 3 Tenside enthaltenden lipophilen Flüssigkeit oder Paste suspendiert sind, wesentlich günstigere Eigenschaften im Vergleich zu den zur gleichen Anwendung bestimmten vorbekannten Mittel haben.

Das für das erfindungsgemäße Mittel verwendete Pfropf-Copolymerisat aus Stärke und hydrolisiertem Polyacrylnitril, dessen Carboxygruppen-Wasserstoffe zu 5 bis 90 % durch Aluminium substituiert sind, kann beispielsweise nach der Lehre des US-Patentes 4,302,369 hergestellt werden, indem man unter den dort angegebenen Bedingungen eine Stärkesuspension mit Acetonitril in Gegenwart eines chemischen Initiators wie Cerammoniumnitrat umsetzt, das entstandene Pfropf-Copolymere mit starken Basen hydrolysiert und dann mit Aluminiumsalzen umsetzt. Das erhaltene Produkt kann dann getrocknet, mit alkoholischer Ammoniaklösung gewaschen und mit Salzsäure auf den gewünschten pH-Wert von 6,0 bis 7,5 eingestellt werden. Andererseits ist es aber auch möglich, dieses Produkt beispielsweise von der Henkel Corporation, Minneapolis z. B. unter der Bezeichnung SGP 157 M käuflich zu erwerben.

Zur Herstellung des erfindungsgemäßen Mittels sind solche Pfropf-Copolymerisate geeignet, die folgende Merkmale aufweisen.

Das Verhältnis der Stärkekomponente zur Summe von Acrylat- und Acrylamidkomponente beträgt im Copolymerisat vorzugsweise 1:3 bis 1:0,9 Gewichtsprozent.

Das Verhältnis von Carboxylgruppen zu Amidgruppen ist vorzugsweise 2:1 bis 9:1 mol:mol.

Im Copolymerisat sind vorzugsweise 25 bis 75 % der Carboxylgruppen durch Aluminium substituiert.

Die Korngröße des Copolymerisats ist vorzugsweise 0,01 bis 0,5 mm.

Als lipophile Flüssigkeiten können Kohlenwasserstoffe, wie zum Beispiel Paraffine, Vaseline, Stearin aber auch pflanzliche und tierische Öle, Wachse oder Fette, wie zum Beispiel Jojoba-Öl, Olivenöl, Erdnußöl, Kokosnußöl, Mandelöl, Sonnenblumenöl, Lanolin, feines Knochenöl, Bienenwachs, Wollfett etc. eingesetzt werden.

Als physiologisch verträgliche Tenside sind sowohl nichtionogene als auch ionogene Tenside geeignet. Als nichtionogene Tenside seien genannt: Lecithine, Lecithinfraktionen und deren Abwandlungsprodukte, Polyoxyethylenfettsäureester wie Polyoxyethylenfettalkoholether, polyoxyethylierte Sorbitanfettsäureester, Glycerin-polyethylenglykoloxystearat, Glycerinpolyethylenglykolrhizinoleat, ethoxylierte Sojasterine, ethoxylierte Rhizinusöle und deren hydrierte Derivate, Cholesterol, Polyoxyethylenpolyoxypropylen-Polymere,
wobei Polyoxyethylenpolyoxypropylen-Polymere mit dem Molgewicht 6800 - 8975, zum Beispiel Pluronic$^{(R)}$ F 68, bevorzugt sind.

Als besonders geeignete Tenside zur Verwendung in den erfindungsgemäßen Mitteln haben sich die in den Patentansprüchen 3 bis 5 gekennzeichneten Substanzen erwiesen.

Polyethylengruppen enthaltende Tenside die zur Herstellung
der Mittel geeignet sind, sind beispielsweise Polyethylenglykole mit einem Molekulargewicht oberhalb ca. 1000 in
Wasser schwer lösliche Polypropylenglykole und Blockpolypolymere beider Verbindungen, wie sie unter dem Namen Pluronic$^{(R)}$ handelsüblich sind. Besonders geeignet ist das unter
der Bezeichnung Pluronic$^{(R)}$ F 68 handelsübliche Tensid, welches
in dem erfindungsgemäßen Mittel in einer Konzentration bis zu
5 Gewichtsprozent bezogen auf das Mittel selbst angewendet wird.
Weitere Polyethylengruppen enthaltende Tenside die sich zur
Herstellung des erfindungsgemäßen Mittels eignen sind beispielsweise die unter dem Namen Cremophor$^{(R)}$ EL handelsüblichen Präparate.

Als Tenside eignen sich auch recht gut Fettalkoholsulfate wie
zum Beispiel das unter der Bezeichnung Lnaette$^{(R)}$ E oder N
handelsübliche Präparat.

Als Fettalkohol oder cholesterinhaltige Tenside die sich zur
Herstellung des erfindungsgemäßen Mittels eignen seien beispielsweise genannt: Stearylalkohol, Palmythylalkohol, Gemische
derselben, wie sie beispielsweise unter der Bezeichnung Lanette$^{(R)}$ O handelsüblich sind oder das im Lanolin enthaltene
Cholesterin.

Da es lediglich auf die Eigenschaften, nicht aber auf die chemische Struktur der Tenside ankommt, sind Mischungen mehrerer Tenside ebenso geeignet wie ein einzelnes definiertes Tensid. Neben den Tensiden enthält das erfindungsgemäße Mittel zusätzlich lipophile Flüssigkeiten oder Pasten die vorzugsweise aus den Komponenten bestehen, die in den Patentansprüchen 6 und 7 gekennzeichnet sind.

Hochsiedende Paraffine die sich als Komponenten für das erfindungsgemäße Mittel eignen sind die in der Galenik üblicherweise verwendeten dünnflüssigen, dickflüssigen, wachsartigen oder festen Paraffine unter anderem auch solche, die unter der Bezeichnung Vaseline$^{(R)}$ handelsüblich sind. Geeignete Stoffe sind auch solche Paraffine, welche mit Wollfettalkoholen emulgiert sind wie die unter dem Namen Eucerin$^{(R)}$ handelsüblichen Produkte.

Für das erfindungsgemäße Mittel geeignete Komponenten sind beispielsweise die pflanzlichen oder tierischen Öle, Wachse oder Fette, welche bereits in der Beschreibung erwähnt wurden.

Aus den einzelnen Komponenten die neben den Pfropf-Copolymeren im erfindungsgemäßen Mittel angewendet werden, werden unter den dem Galeniker wohl bekannten Bedingungen Mischungen hergestellt, die jeweils auf den gewünschten Anwendungsbereich des erfindungsgemäßen Mittels abgestimmt sind.

Zur Herstellung der Mittel werden die Komponenten beispielsweise mittels einer geeigneten Mühle gleichmäßig vermischt und mittels Hitze sterilisiert.

Das erfindungsgemäße Mittel zur Behandlung von Wunden ist bezüglich seiner Zweckbestimmung nicht nur zur Behandlung von Wunden im engeren Sinne geeignet, sondern wie die in der vom Bundesverband der pharmazeutischen Industrie e.v., D-6000 Frankfurt/Main, herausgegebenen "Rote Liste - 1980", aufgeführten
Wundbehandlungsmittel zur Behandlung zahlreicher mit einer
Sekretausscheidung verbundenen Entzündungen oder Verletzungen
der Haut oder Schleimhaut geeignet. Solche Erkrankungen oder
Verletzungen sind beispielsweise Schnittwunden, Stoßwunden,
Rißwunden, Quetschwunden, Brandwunden, Frostwunden, Abschürfungen, Sonnenbrand, Ekzeme, Hämorrhoiden, etc.

Das erfindungsgemäße Mittel zur Behandlung von Wunden kann zusätzlich noch die bei solchen Mitteln üblichen Zusatzstoffe
und Hilfsstoffe (z. B. Duftstoffe) sowie darüberhinaus noch
Wirkstoffe wie sie in diesen Mitteln üblicherweise eingesetzt
werden enthalten. Derartige Wirkstoffe sind z. B. Bakteriostatika (wie zum Beispiel Sulfonamide, wie Sulfadiazin, Sulfatolamid, oder Antibiotika wie Penicilin, Neomycin oder Chloramphenicol), Antimykotika wie z.B. Salicylsäure und deren Derivate wie Silicylhydroxamsäure und Salicylamid oder Miconacol
und Isoconacol und Lokalanesthetika wie z.B. Ester der p-Aminobenzoesäure, wie der Methylester oder Ethylester oder Lidocain
etc.

Das Mittel wird auf die zu behandelnde Stelle des Körpers aufgetragen, und gegebenenfalls mit Gaze abgedeckt.

Das erfindungsgemäße Mittel zeichnet sich durch einen außergewöhnlichen Saugdruck aus und begünstigt demzufolge, daß
die Wunde infolge Wasserentzugs rasch heilt.

Durch die im erfindungsgemäßen Mittel enthaltenen lipophilen
Komponenten und Tenside wird bewirkt, daß die erfindungsgemäßen Mittel wegen des niedrigen Dampfdrucks nicht austrocknen.
Hierdruch wird ein Verkleben von Wunden bzw. Wundrändern vermieden, so daß dieses Mittel problemlos wieder entfernt
werden kann. Darüberhinaus bewirken diese Komponenten, daß
die Zubereitung pastöser wird und somit leichter appliziert
werden kann.

Die nachfolgenden Ausführungsbeispiele dienen zur näheren
Erläuterung des erfindungsgemäßen Mittels.

Beispiel 1

2 g Pfropf-Copolymerisat SGP 157 M der Firma Henkel Corporation, Minneapolis werden mit 0,8 g Pluronic[R] F 68 und 10 g Pur-oba-Öl 30 Minuten lang in einer Kugelmühle vermahlen wobei eine homogene Dispersion entsteht.

Beispiel 2

2 g Pfropf-Copolymerisat SGP 157 M der Firma Henkel Corporation, Minneapolis werden mit 1 g dünnflüssigem Paraffin 5 Minuten lang in einer Kugelmühle vermahlen. Dann setzt man 3 g Paraffin zu, mahlt weitere 5 Minuten lang, setzt nochmals 6 g Paraffin und 1 g Pluronic[R] F 68 zu und mahlt weitere 20 Minuten lang bis eine homogene Dispersion entsteht.

Beispiel 3

2 g Pfropf-Copolymerisat SGP 157 M der Firma Henkel Corporation, Minneapolis mit einer Korngröße von weniger als 0,063 mm und 5 g Pluronic[R] F 68 mit einer Korngröße von weniger als 0,075 mm werden mit 20 g dünnflüssigem Paraffin verrieben. Dann versetzt man die Mischung mit 50 g weißer Vaseline und 25 g dickflüssigem Paraffin und verreibt das Gemisch bis eine gleichmäßige homogene Dispersion entsteht.

Beispiel 4

2 g Pfropf-Copolymerisat SGP 157 M der Firma Henkel Corporation, Minneapolis werden mit 10 g Cremophor [R] EL verrieben. Dann setzt man der Mischung portionsweise 90 g weiße Vaseline zu und verreibt sie bis eine homogene Dispersion entsteht.

Beispiel 5

2 g Pfropf-Copolymerisat SGP 157 M der Firma Henkel Corporation, Minneapolis werden mit 10 g Cremophor$^{(R)}$ EL und 10 g Polyethylenglykol (MG 400) verrieben. Dann setzt man portionsweise
80 g weiße Vaseline zu und verreibt bis eine homogene Dispersion
gebildet ist.

Beispiel 6

2 g Pfropf-Copolymerisat SGP 157 M der Firma Henkel Corporation, Minneapolis mit einer Korngröße kleiner als 0,063 mm und
0,8 g Pluronic$^{(R)}$ mit einer Korngröße kleiner als 0,075 mm werden mit 6 g Cremophor$^{(R)}$ EL verrieben. Dann setzt man portionsweise 36 g weiße Vaseline zu und verreibt bis eine homogene
Dispersion entsteht.

Beispiel 7

2 g Pfropf-Copolymerisat SGP 157 M der Firma Henkel Corporation, Minneapolis mit einer Korngröße kleiner als 0,036 mm und
0,8 g Pluronic$^{(R)}$ F 68 mit einer Korngröße kleiner als 0,075 mm
werden mit 4 g Cremophor$^{(R)}$ EL und 4 g Polyethylenglykol (MG 400)
verrieben. Dann setzt man portionsweise 4,8 g weiße Vaseline
zu und verreibt bis eine homogene Dispersion entsteht.

Beispiel 8

2 g Pfropf-Copolymerisat SGP 157 M der Firma Henkel Corporation, Minneapolis mit einer Korngröße kleiner als 0,063 mm und
0,8 g Pluronic$^{(R)}$ F 68 mit einer Korngröße kleiner als 0,075 mm
werden mit 20 g Eucerin$^{(R)}$ Anhydr. verrieben bis eine homogene
Dispersion entsteht.

Beispiel 9

2 g Pfropf-Copolymerisat SGP 157 M der Firma Henkel Corporation, Minneapolis mit einer Korngröße kleiner als 0,063 mm
und 0,8 g Pluronic[(R)] F 68  mit einer Korngröße kleiner als
0,075 mm werden mit 4 g Cremophor[(R)] EL verrieben. Dann setzt
man portionsweise 16 g Eucerin[(R)] Anhydr. zu und verreibt bis
eine homogene Dispersion entsteht.

Beispiel 10

2 g Pfropf-Copolymerisat SGP 157 M der Firma Henkel Corporation, Minneapolis mit einer Korgröße kleiner als  0,063 mm
und 0,8 g Pluronic[(R)] F 68 mit einer Korngröße kleiner als
0,075 mm werden mit 4 g Cremophor[(R)] EL und 4 g Polyethylenglykol (MG 400) verrieben. Dann setzt man portionsweise 4,8 g
Eucerin[(R)] Anhydr. zu und verreibt bis eine homogene Dispersion
entsteht.

Beispiel 11

2 g Pfropf-Copolymerisat SGP 157 M der Firma Henkel Corporation, Minneapolis mit einer Korngröße kleiner als 0,063 mm
und 0,8 g Pluronic[(R)] F 68 werden mit 20 g Salbengrundlage
zur homogenen Dispersion verrieben.

Die verwendete Salbengrundlage wird wie folgt hergestellt:
35 g dickflüssiges Paraffin, 35 g weiße Vaseline und 30 g Lanette[(R)] N werden bei 90° C aufgeschmolzen und gerührt bis
die Mischung erkaltet ist.

Beispiel 12

2 g Pfropf-Copolymerisat SGP 157 M der Firma Henkel Corporation, Minneapolis mit einer Korngröße kleiner als 0,063 mm
und 0,8 g Pluronic[(R)] F 68 werden mit 20 g Salbengrundlage
zur homogenen Dispersion verrieben.

Die verwendete Salbengrundlage wird wie folgt hergestellt:

6,0 g Wollwachs, 0,5 g Lanette$^{(R)}$ O , 10,0 g dickflüssiges Paraffin und 83,5 g weiße Vaseline werden bei 90° C aufgeschmolzen und gerührt bis die Mischung erkaltet ist.

## Beispiel 13

2 g Pfropf-Copolymerisat SGP 157 M der Firma Henkel Corporation, Minneapolis mit eine Korngröße kleiner als 0,063 mm werden mit 0,8 g Pluronic$^{(R)}$ F 68 mit einer Korgröße kleiner als 0,075 mm und 4 g Cremophor$^{(R)}$ EL verrieben. Dann setzt man portionsweise 16 g der gemäß Beispiel 11 hergestellten Salbengrundlage zu und verreibt bis eine homogene Dispersion entsteht.

## Beispiel 14

Unter den Bedingungen des Beispies 13, jedoch unter Verwendung der gemäß Beispiel 12 hergestellten Salbengrundlage wird eine homogene Dispersion hergestellt.

0099074

- 2 -

<u>Patentansprüche</u>

1. Mittel zur Behandlung von Wunden, dadurch gekennzeichnet,
   daß 2 bis 20 Gewichtsprozent eines Pfropf-Copolymerisats
   aus Stärke und hydrolysiertem Polyacrylnitril, dessen Carboxylgruppen-Wasserstoffe zu 5 bis 90 % durch Aluminium substituiert sind, in einer physiologisch unbedenklichen, 1
   bis 3 Tenside enthaltenden lipophilen Flüssigkeit oder Paste
   suspendiert sind.

2. Mittel zur Behandlung von Wunden gemäß Patentanspruch 1,
   dadurch gekennzeichnet, daß es ein Tensid in einer Konzentration von maximal 5 Gewichtsprozent in einer physiologisch
   unbedenklichen lipophilen Flüssigkeit enthält.

3. Mittel zur Behandlung von Wunden gemäß Patentanspruch 1 und
   2, dadurch gekennzeichnet, daß es als eine Komponente ein
   Polyethylengruppen enthaltendes Tensid enthält.

4. Mittel zur Behandlung von Wunden gemäß Patentanspruch 1 und
   2, dadurch gekennzeichnet, daß es als eine Komponente ein
   Fettalkoholsulfat als Tensid enthält.

5. Mittel zur Behandlung von Wunden gemäß Patentanspruch 1 und
   2, dadurch gekennzeichnet, daß es als eine Komponente einen
   Fettalkohol oder Cholesterin als Tensid enthält.

6. Mittel zur Behandlung von Wunden gemäß Patentanspruch 1 bis
   5, dadurch gekennzeichnet, daß es als eine Komponente ein
   hochsiedendes Paraffin enthält.

7. Mittel zur Behandlung von Wunden gemäß Patentanspruch 1 bis
   5, dadurch gekennzeichnet, daß es als eine Komponente ein
   pflanzliches oder tierisches Öl, Wachs oder Fett enthält.

8. Mittel zur Behandlung von Wunden, gemäß Patentanspruch
   1 bis 7, dadurch gekennzeichnet, daß es zusätzlich ein
   oder mehrere Wirkstoffe in therapeutisch wirksamen Mengen
   enthält.

9. Mittel zur Behandlung von Wunden, gemäß Patentanspruch 8,
   dadurch gekennzeichnet, daß der Wirkstoff ein Bakteriostatikum ist.

10. Mittel zur Behandlung von Wunden gemäß Patentanspruch 8,
    dadurch gekennzeichnet, daß es als Wirkstoff ein Antimykotikum enthält.

11. Mittel zur Behandlung von Wunden gemäß Patentanspruch 8,
    dadurch gekennzeichnet, daß der Wirkstoff ein Lokalanaesthetikum ist.

12. Mittel zur Behandlung von Wunden gemäß Patentanspruch
    3 bis 11, dadurch gekennzeichnet, daß es neben dem Pfropf-
    Copolymeren 2 bis 8 der in den Patentansprüchen gekennzeich-
    neten Komponenten enthält.